# EUROPÄISCHE PATENTSCHRIFT

(11) **EP 0 664 796 B1**
(45) Veröffentlichungstag und Bekanntmachung des Hinweises auf die Patenterteilung: **06.08.1997**
(21) Anmeldenummer: 93922529.8
(22) Anmeldetag: 08.10.1993
(51) Int. Cl.: C07D 233/76, C12P 13/08, C12P 13/10, C07C 229/26

(54) **VERFAHREN ZUR HERSTELLUNG VON AMINOALKYLHYDANTOIN UND AMINOALKYL-ALPHA-AMINOSÄUREN**
METHOD OF PREPARING AMINOALKYLHYDANTOINS AND AMINOALKYL-ALPHA-AMINOACIDS
PROCEDE DE PREPARATION D'AMINOALKYLHYDANTOINE ET D'AMINOALKYL-ALPHA-AMINOACIDES

(30) Priorität: 16.10.1992 DE 4234867
(43) Veröffentlichungstag der Anmeldung: 02.08.1995
(73) Patentinhaber: Degussa Aktiengesellschaft, 60311 Frankfurt (DE)
(72) Erfinder: KOTTENHAHN, Matthias, D-63456 Hanau (DE); DRAUZ, Karlheinz, D-63579 Freigericht (DE); BOMMARIUS, Andreas, D-60323 Frankfurt (DE)
(86) Internationale Anmeldenummer: EP9302753
(87) Internationale Veröffentlichungsnummer: WO9408974

(56) Entgegenhaltungen:
- CHEMIKER-ZEITUNG Bd. 334, Nr. 3 , März 1992 , HEIDELBERG DE Seiten 214 - 218 K. DRAUZ ET AL 'Chemoenzymatische Synthese von N-Carbamoyl-D-4-thialysin und N-Carbamoyl-D-homo-5-thialysin'
- ANGEWANDTE CHEMIE. INTERNATIONAL EDITION. Bd. 30, Nr. 6 , 1991 , WEINHEIM DE Seiten 712 - 714 K. DRAUZ ET AL 'Chemoenzymatische syntheses of omega-ureido D-amino acids' in der Anmeldung erwähnt
- POLISH JOURNAL OF CHEMISTRY Bd. 54, Nr. 9 , 1980 Seiten 1833 - 1840 K. SOBCZYK ET AL '13C NMR spectra of hydantoins and 3-phanyl-2-thiohydantoins of amino acids'

## Beschreibung

Die vorliegende Erfindung betrifft ein Verfahren zur Herstellung von 5-(Aminoalkyl)-hydantoinen der allgemeinen Formel I oder der aus diesen erhältlichen Aminoalkyl-α-aminosäuren der allgemeinen Formel II bzw. deren Salze 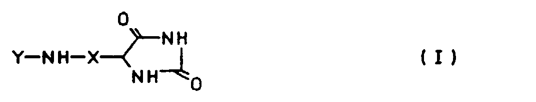 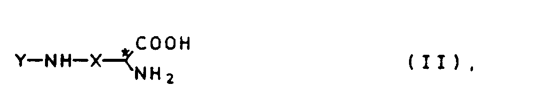 worin
- X: für einen C₁ - C₆-Alkylenrest, der unter Erhalt der benachbarten Aminofunktion substituiert und/oder durch Heteroatome wie z. B. Schwefel oder Sauerstoff unterbrochen sein kann, und
- Y: für Wasserstoff oder einen organischen Rest mit bis zu 8 C-Atomen steht, der unter Erhalt der X und Y benachbarten Aminofunktion substituiert und/oder durch Heteroatome wie z. B. Schwefel oder Sauerstoff unterbrochen sein kann, und
- *: eine am α-C-Atom optisch reine Verbindung bedeutet.

Die Synthese von Aminosäuren über die entsprechenden Hydantoine ist eine bekannte Verfahrensweise. So können z. B. α-Aminosäuren mit Cyanat oder Cyanhydrine mit Ammoniumhydrogencarbonat zum entsprechenden Hydantoin (5-substituierte Imidazolidin-2,4-dione) cyclisiert werden. Die Hydantoine können grundsätzlich mittels chemischer Hydrolyse in die D,L-Aminosäuren, mittels enzymatischer Hydrolyse selektiv in die D- oder L-Aminosäuren gespalten werden. Entsprechende Verfahren sind z. B. aus der DE 39 17 057 C und der EP 0 377 083 A bekannt. Insbesondere die stereospezifische Spaltung der Hydantoine ist besonders interessant, da hierdurch die D-Antipoden natürlicher Aminosäuren herstellbar sind. Wie aus Ch. Syldatk et al. Adv. Biochem. Eng. 44, 29 - 75, 40 (1990) bekannt ist, bereitet die entsprechende Herstellung von Aminosäuren mit geladenen Seitenketten Schwierigkeiten. So fanden auch A. Möller et al., Enzyme Microb. Technol. 1988, 10, 618-625, zwar eine breite Hydantoinase- und Carbamoylase-Aktivität im Bacterium Arthrobacter crystallopoites AM 2, Hydantoine mit geladenen Seitenketten, u. a. auch die Hydantoine des Lysins und des Ornithins, wurden jedoch nicht umgesetzt. Andererseits wird vereinzelt die Umsetzung von basischen Hydantoinen vorgeschlagen (DE 39 17 057 C) bzw. beschrieben (S. Takahashi et al., J. Ferment. Technol. 56, 492 - 8 (1978) und 57, 328 (1979), wobei jedoch im letzteren Fall die Umsetzung nur bis zum Carbamoyl-D-lysin erfolgt. Grundsätzlich hat sich gezeigt, daß - wenn überhaupt - nur die an der Aminofunktion ungeschützten Derivate als Substrate bei der enzymatischen Umsetzung angenommen werden. So werden z. B. entsprechende Amide, wie z. B. 5-(4'-(N-Carbobenzoxyamino)-butyl)-hydantoin mit Agrobacterium radiobacter nicht umgesetzt, während das entsprechend ungeschützte 5-(4'-Aminobutyl)-hydantoin als Substrat erkannt wird. Dies hat zur Folge, daß entsprechend geschützte Aminoalkylhydantoine insbesondere vor einer enzymatischen Umsetzung aufwendig entschützt werden müssen. Die dabei zu verwendenden Schutzgruppen gehören zum Stand der Technik (Barton/Ollis "Comprehensive Organic Chemistry", Vol. 5, S. 333 Pergamon Press Oxford 1979; Chem. Abstr. 110 (1989) 135715x; Chem. Abstr. 109 (1988) 6967m).

Es sind auch Beispiele dafür bekannt (Angew. Chem. 103 (1991) 704 - 706) bestimmte Aminosäuren, wie D-Citrullin, aus L-Aminosäuren gleicher C-Zahl, wie L-Ornithin, herzustellen. Hierzu werden 2 Äquivalente Cyanat mit L-Ornithin umgesetzt, die entstandene α,ω-Bisureidoverbindung wird sauer in das Citrullinhydantoin umgewandelt, welches enzymatisch zu D-Citrullin umgesetzt werden kann. Der vorgeschlagene Syntheseweg funktioniert jedoch nicht mit Aminosäuren, die eine Aminofunktion enthalten.

Aufgabe der vorliegenden Erfindung ist ein Verfahren zur Herstellung von α-Aminosäuren oder 5-substituierten Hydantoinen mit basischer Seitenkette, wobei der bei der Bildung des Hydantoins notwendige Schutz der Aminofunktion in der Seitenkette bezüglich des gesamten Verfahrens nur einen möglichst geringen Aufwand erfordern soll.

Diese Aufgabe wird durch die im Patentanspruch 1 aufgeführten Maßnahmen gelöst.

So betrifft die vorliegende Erfindung ein Verfahren zur Herstellung von 5-(Aminoalkyl)-hydantoinen der allgemeinen Formel I oder der aus diesen erhältlichen Aminoalkyl-α-aminosäuren der allgemeinen Formel II bzw. deren Salze 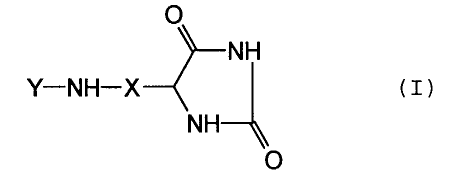 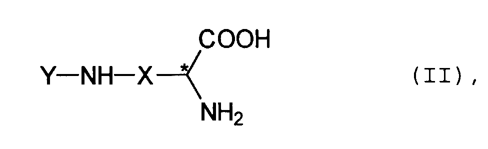 worin
- X: für einen C₁-C₆-Alkylrest, der unter Erhalt der benachbarten Aminofunktion substituiert und/oder durch Heteroatome wie z. B. Schwefel oder Sauerstoff unterbrochen sein kann, und
- Y: für Wasserstoff oder einen organischen Rest mit bis zu 8 C-Atomen steht, der unter Erhalt der X und Y benachbarten Aminofunktion substituiert und/oder durch Heteroatome wie z. B. Schwefel oder Sauerstoff unterbrochen sein kann, und
- *: eine am α-C-Atom optisch reine Verbindung bedeutet,
ausgenommen eine gleichzeitige Heteroatomsubstitution von X und Y, sofern beide Heteroatome mit der X und Y benachbarten Aminofunktion verbunden sind, bei welchem Verfahren man an einer α-Aminosäure der allgemeinen Formel IV oder deren Salz 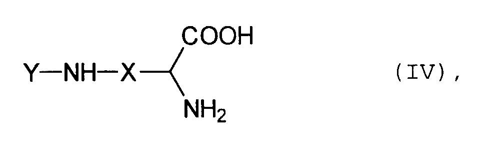 worin
X und Y die oben angegebene Bedeutung haben und als α-Aminosäure oder deren Salz entweder ein Stereoisomerengemisch oder der optische Antipode zu II verwendet wird,
die X und Y benachbarte Aminofunktion unter Erhalt einer Verbindung der allgemeinen Formel III oder deren Salz 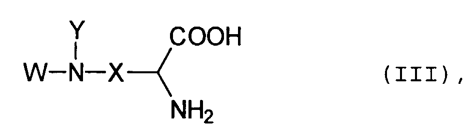 worin
X und Y die oben angegebene Bedeutung haben und W eine mit Säure spaltbare Aminoschutzgruppe bedeutet, mit der mit Säure abspaltbaren Aminoschutzgruppe W schützt, indem die Aminoschutzgruppe W vor der Bildung des Hydantoins (I) eingeführt wird und indem man die daraus resultierende Verbindung der allgemeinen Formel III oder deren Salz mit einem Cyanat umsetzt und durch Säurebehandlung cyclisiert.

Die Bildung des Hydantoinringes ist in der vorliegenden Erfindung an sich in beliebiger Weise möglich, wobei die o. g. Variante ausgehend von der Aminosäure, Umsetzung mit Cyanat und Cyclisieren durch Säureeinwirkung bevorzugt wird, da hierbei direkt das an der Aminofunktion entschützte Hydantoin erhalten wird. Die Hydantoinbildung ist lediglich insoweit limitiert, als daß eine Säurebehandlung bei der Umsetzung erst zu einem Zeitpunkt im Gesamtverfahren erfolgen darf, zu dem eine Aminofunktion nicht mehr an Konkurrenzreaktionen teilnehmen kann. Dies ist beim Verfahren ausgehend vom Cyanhydrin und auch beim bevorzugten Verfahren, der Umsetzung mit dem Cyanat, gegeben. Der Umstand, daß beim bevorzugten Verfahren bei der sauren Cyclisierung gleichzeitig die Aminoschutzgruppe abgespalten wird, ist von besonderem Vorteil, da hierdurch die bislang aufwendige Entschützung völlig entfällt. Obwohl im vorliegenden Verfahren Verfahrensschritte mit Säurebehandlung enthalten sein können, ist erfindungsgemäß keine selektiv abspaltbare Schutzgruppe, wie z. B. die Benzyloxycarbonyl-Schutzgruppe, für den Schutz der X benachbarten Aminofunktion notwendig.

Zur Darstellung der Aminoalkyl-α-aminosäure kann das erhaltene Hydantoin wie eingangs beschrieben weiter umgesetzt werden. Bevorzugt ist hierbei die enzymatische Spaltung zu den stereospezifischen Aminosäuren oder carbamoylierten Aminosäuren, da die chemische Hydantoinspaltung zum Racemat führt. Besonders vorteilhaft ist hierbei auch, daß unter bestimmten (alkalischen) Bedingungen das vom Enzym nicht umgesetzte Hydantoin racemisiert und hierdurch ein vollständiger Umsatz zur Aminosäure erreicht wird. Mit dem vorliegenden Verfahren lassen sich insbesondere auch D-Antipoden natürlicher Aminosäuren, wie z. B. D-Lysin oder D-Ornithin, sowie auch die Antipoden nichtnatürlicher Aminosäuren wie Thialysin, Oxalysin, Abiziin und weitere erhalten. Diese Aminosäuren finden zunehmend Verwendung im Pharmabereich und als Racematspaltagentien. Sie werden nicht oder nur in geringem Maße vom Körper metabolisiert und eignen sich damit zur Erhöhung der Stabilität von Pharmaka. Als Racematspaltagentien können z. B. Polymere des 0-Lysins verwendet werden (Lahav et al., Reactive Polymers 6, 241 - 253 (1987).

Wie bereits ausgeführt, kann die Hydantoinsynthese gemäß der vorliegenden Erfindung erfolgen durch Umsetzen einer Verbindung der allgemeinen Formel III, 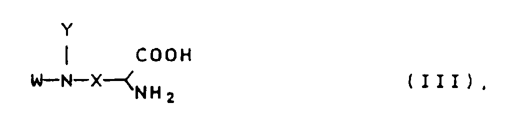 worin
- W: eine mit Säure spaltbare Aminoschutzgruppe, wie z. B. Trifluoracetyl oder t-Butyloxycarbonyl bedeutet und
- X und Y: die oben angegebene Bedeutung haben,
mit Cyanat zur Carbamoylaminosäure, die anschließend in einem sauren Verfahrensschritt zum Hydantoin zyklisiert wird. Hierbei wird gleichzeitig die Schutzgruppe W abgespalten und die Verbindung der allgemeinen Formel I erhalten.

Die Schutzgruppe W ist notwendig, da sonst die Aminofunktion bei der Umsetzung mit Cyanat ebenfalls carbamoyliert würde. Die erfindungsgemäße Verfahrensweise vermeidet einen zusätzlichen aufwendigen Reaktionsschritt, Katalysatorkosten und weiteren Abfall, da die üblicherweise für die Hydantoinbildung eingesetzte Benzyloxycarbonyl-Schutzgruppe durch Hydrierung entfernt wird.

Bevorzugt steht in den obigen allgemeinen Formeln Y-NH-X- für H₂N-(C₁ - C₆)-Alkyl-; insbesondere D-Lysin und D-Ornithin lassen sich mit der vorliegenden Erfindung günstig herstellen.

Die Erfindung wird anhand eines Schemas und in den nachfolgenden Beispielen näher ausgeführt.

Das Schema zeigt beispielhaft die Darstellung von D-Lysin ausgehend vom L-Lysin.

### Beispiel 1

### Synthese von 5-(4'-Aminobutyl)-L-hydantoin

24,2 g (0,1 mol) N^{ε}-Trifluoracetyl-L-lysin werden in 100 ml Wasser suspendiert. Es werden 7,15 g ( 0,11 mol) NaOCN zugesetzt und für 4 h auf 70 - 75°C erwärmt. Danach wird auf 40°C abgekühlt und mit konz. HCl auf pH 4 - 5 gestellt. Nun werden 16,5 ml konz. HCl zugesetzt und 4 h unter Rückfluß gekocht. Die Lösung wird nach dem Abkühlen über einen sauren Ionentauscher (Merck IR 120, H⁺-Form) filtriert und anschließend mit wässr. Ammoniak eluiert. Dieses Eluat wird im Vakuum weitgehend eingedampft und mit Ethanol (50 ml) versetzt. Der dabei ausfallende Feststoff wird filtriert und im Vakuum getrocknet.
Ausbeute 13,95 g (82 % d. Th.)

### Beispiel 2

### Umsetzung von 5-(4'-Aminobutyl)-L-hydantoin mit Agrobacterium radiobacter

4 g 5-(4'-Aminobutyl)-L-hydantoin werden in einem Druckgefäß in 66 ml Wasser gelöst. Durch Zusatz von HCl wird ein pH-Wert von 8,2 - 8,3 eingestellt. Nach Zusatz von 6,5 g Biomasse (Agrobacterium radiobacter; Recordati/De.Bi, Mailand, Italien) wird mit N₂ entgast und ein Überdruck von 2 - 4 bar eingestellt. Bei 40°C läßt man 48 h reagieren, zentrifugiert die Biomasse ab und klärt das Zentrifugat bei pH 6 mit Aktivkohle. Anschließend wird an einem sauren Ionenaustauscher (Merck IR 120, H⁺-Form) entsalzt. Das ammoniakalische Eluat wird im Vakuum auf ein Restvolumen von 10 ml eingedampft, mit HCl auf pH 2 gestellt und mit 40 ml Ethanol versetzt.
Ausbeute: 3,7 g D-Lysin Hydrochlorid (87 % d. Th.)

### Beispiel 3 (Vergleich)

### Umsetzung von 5-(4'-(N-Carbobenzoxy-amino)-butyl)-L-hydantoin mit agrobacterium radiobacter

4 g 5-(4'-(N-Carbobenzoxy-amino)-butyl)-L-hydantoin wurden analog Beispiel 2 umgesetzt.
Es wurde ausschließlich Edukt isoliert, es fand keine Umsetzung statt.

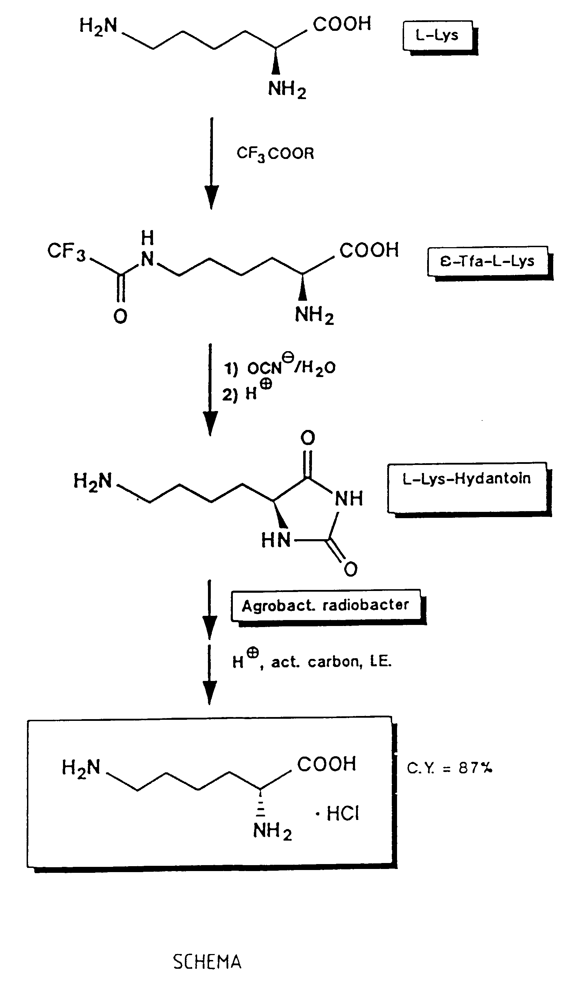

## Patentansprüche

1. Verfahren zur Herstellung von 5-(Aminoalkyl)hydantoinen der allgemeinen Formel I oder der aus diesen erhältlichen Aminoalkyl-α-aminosäuren der allgemeinen Formel II bzw. deren Salze 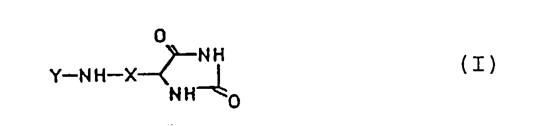 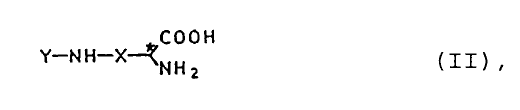 worin
X für einen C₁-C₆-Alkylrest, der unter Erhalt der benachbarten Aminofunktion substituiert und/oder durch Heteroatome wie z. B. Schwefel oder Sauerstoff unterbrochen sein kann, und
Y für Wasserstoff oder einen organischen Rest mit bis zu 8 C-Atomen steht, der unter Erhalt der X und Y benachbarten Aminofunktion substituiert und/oder durch Heteroatome wie z. B. Schwefel oder Sauerstoff unterbrochen sein kann, und
* eine am α-C-Atom optisch reine Verbindung bedeutet,
ausgenommen eine gleichzeitige Heteroatomsubstitution von X und Y, sofern beide Heteroatome mit der X und Y benachbarten Aminofunktion verbunden sind, bei welchem Verfahren man an einer α-Aminosäure der allgemeinen Formel IV oder deren Salz 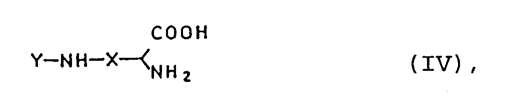 worin
X und Y die oben angegebene Bedeutung haben und als α-Aminosäure oder deren Salz entweder ein Stereoisomerengemisch oder der optische Antipode zu II verwendet wird,
die X und Y benachbarte Aminofunktion unter Erhalt einer Verbindung der allgemeinen Formel III oder deren Salz 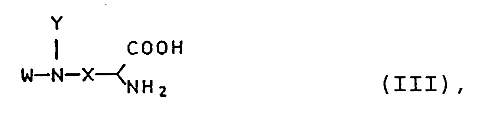
worin
X und Y die oben angegebene Bedeutung haben und
W eine mit Säure spaltbare Aminoschutzgruppe bedeutet, mit der mit Säure abspaltbaren Aminoschutzgruppe W schützt,
**dadurch gekennzeichnet**,
daß die Aminoschutzgruppe W vor der Bildung des Hydantoins (I) eingeführt wird und
daß man die daraus resultierende Verbindung der allgemeinen Formel III oder deren Salz mit einem Cyanat umsetzt und durch Säurebehandlung cyclisiert.

2. Verfahren nach Anspruch 1,
**dadurch gekennzeichnet**,
daß die Abspaltung der Aminoschutzgruppe W mit der Säurebehandlung erfolgt.

3. Verfahren nach Anspruch 2,
**dadurch gekennzeichnet**,
daß man als mit Säure spaltbare Aminoschutzgruppe W Trifluoracetyl oder t-Butyloxycarbonyl verwendet.

4. Verfahren nach einem der vorhergehenden Ansprüche,
**dadurch gekennzeichnet**,
daß das Hydantoin enzymatisch in die Aminosäure oder carbamoylierte Aminosäure gespalten wird.

5. Verfahren nach einem der vorhergehenden Ansprüche,
**dadurch gekennzeichnet**,
daß Y-NH-X- für H₂N-(C₁ - C₆)-Alkyl- steht.

## Claims

1. A method for the preparation of 5-(aminoalkyl)hydantoins corresponding to the general formula I or of the aminoalkyl-α-amino acids obtainable from these corresponding to the general formula II or their salts 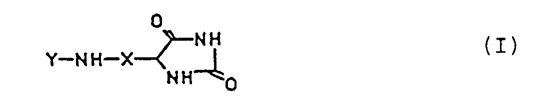 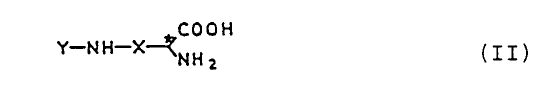 wherein
X represents a C₁-C₆ alkyl group which, with preservation of the adjacent amino function, can be substituted and/or interrupted by hetero atoms such as, for example, sulphur or oxygen, and
Y represents hydrogen or an organic group having up to 8 C atoms which, with preservation of the amino function adjacent to X and Y, can be substituted and/or interrupted by hetero atoms such as, for example, sulphur or oxygen, and
* denotes a compound optically pure at the α-C atom,
excluding a simultaneous hetero atom substitution of X and Y, if both hetero atoms are bonded to the amino function adjacent to X and Y, in which method, in an α-amino acid corresponding to the general formula IV or salt thereof 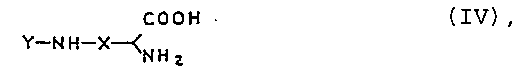 wherein
X and Y have the meaning given above and the α-amino acid or salt thereof used is either a mixture of stereoisomers or is the optical antipode to II, the amino function adjacent to X and Y is protected by an amino-protecting group W which can be split off by acid, with preservation of a compound corresponding to the general formula III or salt thereof 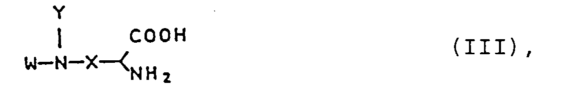 wherein
X and Y have the meaning given above and W denotes an amino-protecting group which can be split off by acid,
characterised in that
the amino-protecting group W is introduced prior to the formation of the hydantoin (I) and in that the resulting compound corresponding to the general formula III or salt thereof is reacted with a cyanate and cyclised by treatment with acid.

2. The method according to claim 1, characterised in that the splitting off of the amino-protecting group W is effected by the treatment with acid.

3. The method according to claim 2, characterised in that trifluoroacetyl or t-butyloxycarbonyl are used as the amino-protecting group W which can be split off by acid.

4. The method according to one of the preceding claims, characterised in that the hydantoin is split enzymatically into the amino acid or carbamoylated amino acid.

5. The method according to one of the preceding claims, characterised in that -Y-NH-X- represents H₂N-(C₁ - C₆)-alkyl-.

## Revendications

1. Procédé de préparation de 5-(aminoalcoyl)-hydantoïnes de formule générale I ou des aminoalcoyl-α-aminoacides que l'on peut obtenir à partir de ceux-ci de formule générale II ou de leurs sels, 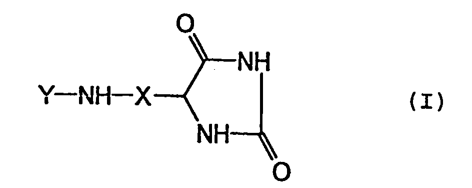 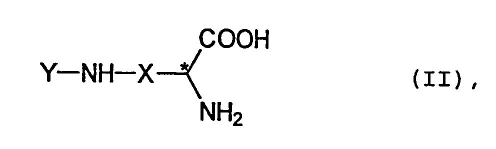 dans lesquelles
X représente un radical alcoyle en C₁-C₆ qui peut être substitué tout en conservant la fonction amine voisine et/ou interrompu par des hétéroatomes comme par exemple du soufre ou de l'oxygène, et
Y représente de l'hydrogène ou un radical organique ayant jusqu'à 8 atomes de C, qui peut être substitué tout en conservant la fonction amine voisine de X et de Y et/ou interrompu par des hétéroatomes comme par exemple le soufre ou l'oxygène, et
* signifie un composé optiquement pur sur l'atome de C en α,
à l'exception d'une substitution simultanée par des hétéroatomes, de X et de Y, pour autant que les deux hétéroatomes soient reliés à la fonction amine voisine de X et de Y, procédé dans lequel on utilise sur un α-aminoacide de formule générale IV ou un de ses sels, 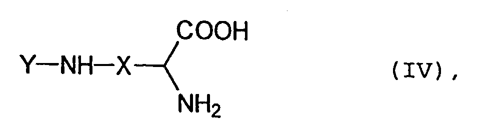 dans laquelle X et Y ont la signification indiquée ci-dessus, et comme α-aminoacide ou un de ses sels, soit un mélange de stéréo-isomères ou les antipodes optiques à II, on protège la fonction amine voisine de X et de Y tout en obtenant un composé de formule générale III ou un de ses sels, 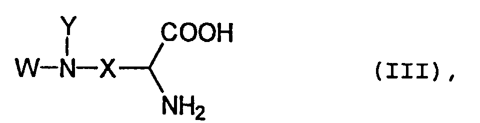 dans laquelle X et Y ont les significations fournies ci-dessus, et
W signifie un groupe de protection pour amine, clivable par des acides avec le groupe de protection pour amine W, clivable par des acides,
caractérisé en ce que
l'on introduit le groupe de protection pour amine W avant la formation de l'hydantoïne (I) et en ce que le composé qui en résulte de formule générale III ou un de ses sels, est mis à réagir avec un cyanate et on cyclise par traitement par un acide.

2. Procédé selon la revendication 1,
caractérisé en ce que
le clivage du groupe de protection pour amine W s'effectue par traitement par un acide.

3. Procédé selon la revendication 2,
caractérisé en ce que
l'on utilise comme groupe de protection pour amine clivable avec des acides W, le trifluoroacétyle ou le terbutoxycarbonyle.

4. Procédé selon l'une des revendications précédentes,
caractérisé en ce que
l'on clive l'hydantoïne par voie enzymatique en l'aminoacide ou en aminoacide carbamoylé.

5. Procédé selon l'une des revendications précédentes,
caractérisé en ce que
Y-NH-X représente H₂N-(alcoyle en C₁-C₆).
